# EUROPEAN PATENT APPLICATION

(11) **EP 2 455 119 A1**
(43) Date of publication of application: **23.05.2012**
(21) Application number: 12156033.8
(22) Date of filing: 06.04.2010
(51) Int. Cl.: A61M 5/172, A61M 5/142, A61B 5/00, A61B 5/145

(54) **Metabolite management system**

(30) Priority: 02.04.2009 US 166052 P; 18.05.2009 US 179017 P
(62) Divisional of application: 10720313.5
(71) Applicant: G-Sense Ltd., 39032 Tirat Hacarmel (IL)
(72) Inventor: Shekalim, Avraham, 36842 Nesher (IL); Peleg, Noam, 19351 Gan-Ner (IL); Zeltser, Carmel, 40200 Kfar Vitkin (IL)
(74) Representative: Modiano, Micaela Nadia

(57) **Abstract**

A metabolite monitoring system configured to deliver drugs and obtain metabolite samples via one common skin perforation and measure metabolite content by way of an optical measuring-cell having a primary flow-path of constant cross-sectional area.

## Description

### FIELD AND BACKGROUND OF THE INVENTION

The present invention generally relates to an automated combined drug-delivery and metabolite-monitoring system and, particularly, relates to two primary of aspects each believed to be patentable significance in its own right and which are most preferably used in synergy to provide a particularly advantageous combination.

The first aspect relates to a combined continuous subcutaneous drug-infusion and metabolite-measurement system in which the drug delivery and metabolite sampling via one skin perforation common to both a cannula and a microdialysis probe. It is well-known that insulin delivery and blood-glucose sampling, for example, are performed at points removed from each other so that the sampling will be representative of the overall glucose content and not just representative of the local, insulin rich region. Generally, manufactures of such systems recommend that the sampling probe be inserted into the patient's body at least ten centimeters away from the cannula as shown below:
"For users who wear an insulin pump, make sure that the sensor insertion site is at least two (2) inches away from the insulin infusion site. Users who inject insulin should be instructed to administer injections at least three (3) inches away from the Sensor insertion site." Medtronic MiniMed: Important Safety Information [online article 2007] http://www.minimed.com/about/safety.html.

A patient using these systems must therefore pierce the skin to insert the cannula and again to insert the sampling probe and again twice when replacing them. Furthermore, these guidelines restrict the degree of miniaturization needed to make the system more inconspicuous and comfortable for the wearer.

Therefore, it would advantageous to a user to have a system reducing the required number of skin piercing to an absolute minimum and facilitating further miniaturization.

A second aspect of the present application relates to an optical measuring-cell in a metabolite-monitoring unit. Particularly, it relates to improving reliability of the optical measurement by ensuring a steady flow of uniform mixture of dialysate and reagent through the section of the measuring-cell in which optical measurements are obtained. It is well-known in optical analytical methods that a beam of light is directed through a dialysate sample in a flow path for the sake of capturing transmission or reflectance data indicative of the dialysate content of the sample. The optical measurements are generally obtained as the sample flows through a measuring segment having an enlarged diameter ensuring a sufficient amount of sample is exposed to the light beam to produce optical data indicative of a metabolite content. However, the resulting pressure drop creates an undesirable situation in which sample tends to become lodged in the measuring segment. Future optical readings are consequently compromised because they are based on the entire volume of illuminated sample including the outdated dialysate sample. Additional complications arise from the enlarged diameter of the flow path when air bubbles inadvertently enter the system and also tend to become lodged in the measuring-cell. Their presence creates gas/liquid interfaces that refract the light beam unpredictably, again compromising data integrity. Furthermore, they render an algorithm for converting the optical data into a metabolite-content value ineffective because the conversion is based on a predefined dialysate volume which, in the presence of air bubbles, is incorrect.

Therefore, there is a need for an optical measuring-cell having a flow path ensuring a steady flow of uniform mixture of dialysate and reagent to provide enhanced reliability when obtaining optical measurements of metabolite samples.

### SUMMARY OF THE INVENTION

The present invention is a combined drug-delivery and metabolite-monitoring system.

According to the teachings of the present invention there is provided, a system for managing a metabolite in a body of a patient comprising:(a) a drug-delivery unit including a cannula configured for insertion into the body, and (b) a metabolite-monitoring unit including a sampling probe configured for insertion into the body, the drug-delivery unit and the metabolite-monitoring unit being disposed in a housing such that the cannula and the sampling probe are in close proximity to each other thereby enabling their insertion into the body via one common skin perforation.

According to a further feature of the current invention the sampling probe is threaded through at least one perforation in a wall of the cannula.

According to a further feature of the current invention there is also provided a control unit deployed to control the drug-delivery unit and the metabolite-monitoring unit, the control unit being configured to: (a) actuate the drug-delivery unit to deliver separate dosages of a drug to the patient, each of the dosages being separated by an interval of time, and (b) actuate the metabolite-monitoring unit to obtain a metabolite sample from the patient after a minimum time delay following any one of the dosages sufficient for the metabolite sample to be indicative of an overall metabolite content in the body of the patient.

According to a further feature of the current invention the sampling probe is selected from the group consisting of a microdialysis fiber, electrochemical probe, fiber-optic sensor, and fiber-coupled fluorescence affinity sensor.

According to a further feature of the current invention the metabolite-monitoring unit includes a layered, optical-measuring-cell.

According to a further feature of the current invention the layered, optical-measuring-cell includes a primary flow path of constant cross-sectional area.

There is also provided according to the teachings of the present invention a metabolite in a body of a patient comprising: (a) providing a combined drug delivery and metabolite measurement system having a cannula and a metabolite sampling probe disposed in close proximity to each other so as to enable their insertion into the body via one common skin perforation, and (b) inserting the cannula and the metabolite sampling probe into the body via one common skin perforation.

According to a further feature of the current invention there is also provided:
(a) delivering separate dosages of a drug to the patient, each of the dosages being separated by an interval of time, (b) obtaining a metabolite sample from the patient after a minimum time delay following any one of the dosages sufficient for the metabolite sample to be indicative of an overall metabolite content in the body of the patient.

According to a further feature of the current invention the metabolite sampling probe includes a looped microdialysis sampling probe.

According to a further feature of the current invention the metabolite-measurement system includes a layered optical measuring-cell.

According to a further feature of the current invention the layered optical measuring-cell includes a primary flow path having a substantially constant cross-sectional area between any two points along the flow path.

According to a further feature of the current invention the metabolite sampling probe includes a looped microdialysis sampling probe.

There is also provided according to the teachings of the present invention (a) providing a combined drug delivery and metabolite measurement system, (b) delivering separate dosages of a drug to the patient, each of the dosages being separated by an interval of time, and (c) obtaining a metabolite sample from the patient after a minimum time delay following any one of the dosages sufficient for the metabolite sample to be indicative of an overall metabolite content in the body of the patient.

According to a further feature of the current invention the combined drug delivery and metabolite measurement system includes a cannula and a metabolite sampling probe disposed in close proximity to each other so as to enable their insertion into the body via one common skin perforation.

According to a further feature of the current invention there is also provided inserting the cannula and the metabolite sampling probe into the body via one common skin perforation.

According to a further feature of the current invention themetabolite sampling probe includes a looped microdialysis sampling probe.

According to a further feature of the current invention themetabolite-measurement system includes a layered optical measuring-cell.

According to a further feature of the current invention the layered optical measuring-cell includes a primary flow path having a substantially constant cross-sectional area between any two points along the flow path.

According to a further feature of the current invention the metabolite sampling probe includes a looped microdialysis sampling probe.

There is also provided according to the teachings of the present invention an optical measuring-cell for use with a metabolite monitoring unit that employs an optical sensor with a light source deployed for illuminating a measurement region, the optical measuring-cell comprising:(a) a sheet of material having a groove defining a flow path passing through the measurement region illuminated by the light source, a portion of the groove within the measurement region being configured in a geometrical arrangement having a length at least 1.5 times greater than a maximum dimension of the measurement region; and (b) at least one sheet of transparent material bonded to the sheet of material so as to enclose the groove defining a flow path thereby ensuring sufficient liquid is exposed to the light source to facilitate accurate optical measurements.

According to a further feature of the current invention the at least one sheet of transparent material is implemented as two sheets, each of the two sheets being bonded to opposing faces of the sheet of wherein the groove defining a flow path is at least partially implemented as a slot.

According to a further feature of the current invention the flow path includes a flow path having a substantially constant cross-sectional area not varying more than 20% between any two points along the length of the flow path.

According to a further feature of the current invention the sheet of material includes a sheet of aluminum.

According to a further feature of the current invention the geometric arrangement includes a plurality of curves.

According to a further feature of the current invention the geometric arrangement includes a spiral.

According to a further feature of the current invention the length of the flow path is at least two times greater than a maximum dimension of the area illuminated by the light source.

According to a further feature of the current invention the length of the flow path is at least 3 times greater than a maximum dimension of the area illuminated by the light source.

According to a further feature of the current invention the groove extends outside the area illuminated by a light source so as to define a flow path upstream from the area illuminated by the light source to enable uniform mixing of dialysate and reagent prior to exposure to the light source.

There is also provided according to the teachings of the present invention a layered, optical measuring-cell partially illuminated by a light source for a metabolite-monitoring unit comprising: (a) a sheet of material having a groove defining an illuminated flow-path segment in fluid connection with a non-illuminated flow-path segment wherein a cross-sectional area of the illuminated flow-path segment substantially matches a cross-sectional area of the non-illuminated flow-path segment thereby facilitating uniform flow from the non-illuminated, flow-path segment to the illuminated, flow-path segment.

According to a further feature of the current invention the the illuminated flow-path segment has a geometrical arrangement of a length at least 1.5 times greater than a maximum dimension of an area of illuminated by the light source.

According to a further feature of the current invention the illuminated flow-path segment has a geometrical arrangement of a length at least two times greater than a maximum dimension of an area of illuminated by the light source.

According to a further feature of the current invention the illuminated flow path-segment has a geometrical arrangement of a length at least three times greater than a maximum dimension of an area of illuminated by the light source.

According to a further feature of the current invention the geometric arrangement includes a plurality of curves.

According to a further feature of the current invention the geometric arrangement includes a spiral.

According to a further feature of the current invention the illuminated flow-path segment being disposed in a first plane and the non-illuminated flow-path segment being disposed in a second plane wherein the first plane is different from the second plane.

### BRIEF DESCRIPTION OF THE DRAWING

The invention is herein described, by way of example only, with reference to the accompanying drawings, wherein:
FIG. 1 is a schematic, block diagram depicting a combined continuous drug-delivery and metabolite-measurement system.
FIG. 2 is an isometric, bottom-view of the system of FIG. 1 depicting a cannula and sampling probe arrangement.
FIG. 3 is a schematic, enlarged, cross-sectional view of the cannula and sampling probe arrangement of FIG 2.
FIG. 4 is an isometric, overall view of a combined cannula and metabolite probe insertion mechanism.
FIGS. 5-6 are isometric, enlarged views of the slotted portion of the insertion mechanism of FIG. 4 in pre-insertion and insertion states, respectively.
FIG. 7 is a schematic, cross-sectional view of the insertion mechanism in a loaded, pre-insertion state.
FIG. 8 is a flow chart depicting the sequence of operations employed to provide the synchronization of drug-delivery and metabolite-sampling.
FIGS. 9-11 are comparative time lines depicting synchronization between drug-delivery and metabolite-sampling.
FIG. 12 is an isometric, exploded-view of a three-layered optical measuring-cell.
FIG. 13 is a schematic, top view of a layered optical measuring-cell depicting flow paths and associated inlets and outlets.
FIG. 14 is an isometric, exploded view of a twa-layered optical measuring-cell.
FIG. 15 is a schematic top view of a two-layered optical measuring-cell.
FIG. 16 is an isometric, cross-sectional view of a two-layered optical measuring-cell along line X-X of FIG. 15.
FIG. 17 is an isometric, top view of a layered, optical measuring-cell depicting flow paths and associated inlets and outlets in a bent, deployment state.
FIG. 18 is an isometric, exploded-view of a combination drug-delivery and metabolite monitoring system depicting the optical measuring-cell, system housing, seals and cannula prior to assembly.
FIG. 19 is an isometric, top-view of an assembled combination drug-delivery and metabolite monitoring system.
FIG. 20 is schematic top view of a combination drug-delivery and metabolite monitoring system.
FIG. 21 is schematic, cross-sectional view of the combination drug-delivery and metabolite-monitoring system along line Y-Y of FIG. 20.
FIG. 22 is a schematic, enlarged, cross-sectional view of the combination drug-delivery and metabolite-monitoring system depicting the interface between liquid reservoirs and optical measuring-cell of the metabolite probe to the optical measuring-cell.
FIG. 23 is a schematic, enlarged, cross-sectional view of the metabolite probe and its connection to the system housing while deployed in tissue.
FIG. 24 is a schematic, cross-sectional side-view of the metabolite-management system depicting an alternative embodiment of a cannula/probe arrangement in a deployment state.
FIG. 25 is a schematic, cross-sectional bottom view along line XX-XX of Figure 25.
FIG. 26 is a schematic, cross-sectional side-view of the metabolite-management system depicting an alternative embodiment of a cannula/probe arrangement in a post deployment state.
FIG. 27 is a schematic, cross-sectional bottom view along line XX-XX of Figure 26.

### DESCRIPTION OF THE PREFERRED EMBODIMENTS

The present invention is a combined drug-delivery and metabolite-monitoring system.

The principles and operation of the combined drug-delivery and metabolite-monitoring system according to the present invention may be better understood with reference to the drawings and the accompanying description.

The present invention includes two primary aspects, each of which is believed to be of patentable significance in its own right, and which are most preferably used in synergy to provide a particularly advantageous combination, as will be come clear. A first aspect of the invention relates deployment of cannula and metabolite microdialysis probe by way of one common skin perforation for reducing the number of skin pricks a patient must suffer when deploying automated drug-delivery and metabolite systems. A second aspect of the present invention relates to an optical measuring-cell having a flow path of constant cross-sectional area for improving reliability as will be discussed. Before addressing these aspects of the present invention, it should be noted that aspects of the invention relating to the optical measuring-cell also relate to stand-alone metabolite-monitoring systems; but, as a matter of convenience will be described in the context of a combination drug-delivery and metabolite monitoring system.

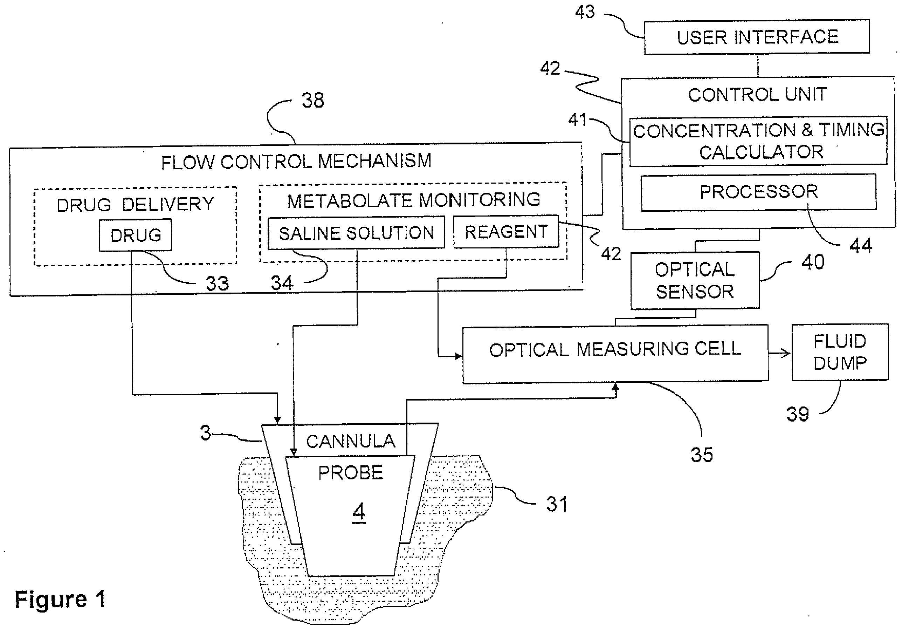

Turning now to the figures, Figure 1 illustrates schematically a metabolite-management system for drug-delivery and in vivo metabolite concentration monitoring within a body fluid.

Cannula 3 and microdialysis probe 4 are introduced into a tissue 31 of the subject through one common skin perforation. A quantity of drug is delivered from drug reservoir 33 and at the appropriate time, as will be discussed, saline is supplied from a saline reservoir 34 to probe 4, where it absorbs metabolite from the surrounding body fluids through the membrane. The dialysate is fed into optical measuring-cell 35 where dialysate is mixed with reagent supplied from reservoir 42 and the concentration of metabolite in the dialysate is determined by way of optical measurements obtained by an optical sensor 40 sensing color change resulting from a chemical reaction of the dialysate metabolite and the reagent.

Flow of the drug to cannula 3, saline to probe 4, reagent and metabolite-rich dialysate to optical measuring-cell 35 are all controlled by a flow control mechanism 38. The dialysate and reagent mix in optical measuring-cell 35, and displace previously sampled fluid into a fluid dump 39. Flow control mechanism 38 may be any suitable flow control mechanism, including but not limited to, various active displacement pump systems, and various arrangements of valves controlling flow from pressurized reservoirs, all as are known in the art. One particularly preferred but non-limiting example of a suitable flow control mechanism may be found in PCT Patent Application Publication No. WO 2008/056363, which is hereby incorporated in its entirety as set out therein.

Operation of flow control mechanism 38 and processing of outputs from optical sensor 40 are controlled by a control unit 42 including a concentration and timing calculator 41 and a processor 44. A local or remote user interface 43 enables a user to input system parameters such as dosage rates and other information used in the calculation of metabolite concentration, sampling timing, and dosing delays.

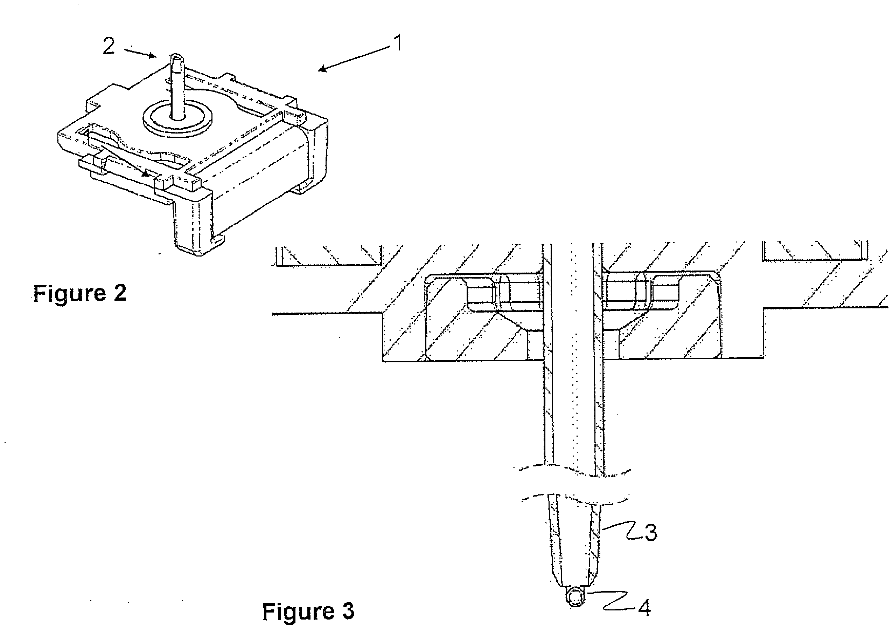

Turning now to the first aspect of the present invention, Figures 2 and 3 depict a combined drug-delivery and metabolite-monitoring system 1, and specifically, depict a cannula and metabolite-probe arrangement 2 facilitating insertion into a patient's body via one common skin perforation as noted above. In a non-limiting, exemplary embodiment, metabolite-probe 4 is disposed at the distal end of cannula 3 as shown in Figure 2; however, it should be noted that any configuration in which probe 4 and cannula 3 are disposed in close proximity to each other enabling their insertion via a common skin perforation is included within the scope of the present invention. For the purposes of this document, "skin perforation" refers to an opening in the skin, typically roughly circular, with a diameter typically between 0.3-1.5 millimeters, and in some cases, up to about 2 millimeters in diameter.

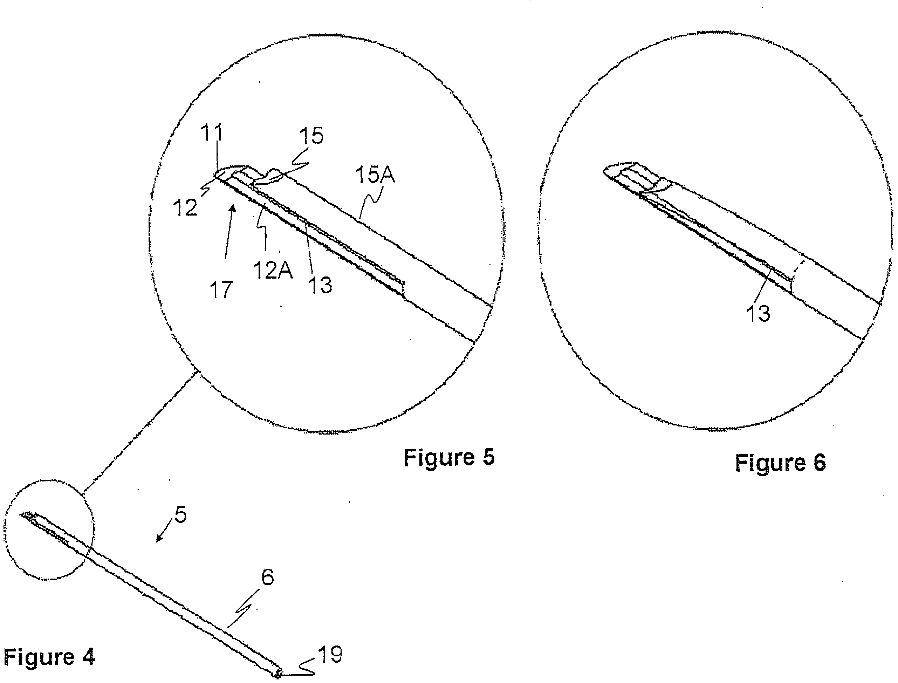

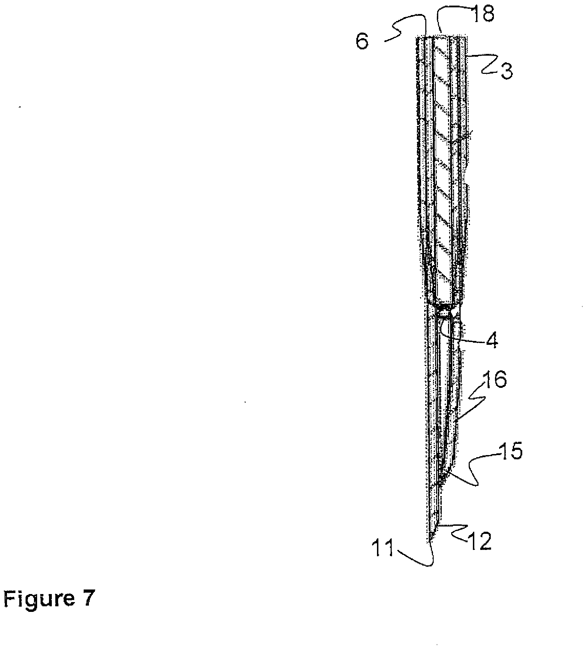

Figures 4-7 depict a non-limiting, exemplary embodiment of inserter mechanism 5 configured to deploy probe cannula 3 and probe 4 at the desired tissue depth. Insertion mechanism 5 includes an elongated shaft 6 implemented as a tube, a penetrating tip 11 for piercing the skin and tissue, a beveled end 12, a slot 13 for receiving probe 4 and a push rod 18 (most clearly shown in Fig. 7) serving as the actuator element for actuating the release of probe 4 from insertion tube 6. Penetrating tip 11, in a non-limiting exemplary embodiment, is implemented as a cutting edge formed by a single bevel, but it should be appreciated that a cutting edge or a puncturing point formed by a taper are also included within the scope of any embodiment of the current invention. Slot 13 is disposed across beveled end 12 so as to divide beveled end 17 into two beveled surfaces, 12 and 15. Slot sides 12A and 15A straddling slot 13 are resiliently biased to mutually bear on each other to avoid catching tissue in slot 13 during penetration into the body tissue and to help retain probe 4 within slot 13 prior to deployment. Slot 13 is disposed in a manner that preserves the structural integrity of penetrating tip 11, and has a depth of three to four millimeters in a non-limiting embodiment. Push rod 18 is disposed in the inserter tube lumen 19 (most clearly shown in Figure 4) in a manner providing freedom of axial movement and inserter mechanism 5 and push rod 18 are loaded into cannula 3.

Prior to deployment, probe 4 is inserted into slot 13, where it is retained by the closing together of two sides of slot 13 and/or by maintaining slight tension in the probe fiber. The non-beveled end of insertion mechanism 5 carrying probe 4 is inserted through the leading end of cannula 3 into its lumen (not shown). Cannula 3 and insertion mechanism 5 carrying probe 4 and containing push rod 18 are all inserted into the subject's body, either manually or by any suitable automated insertion mechanism.

Upon achieving the desired deployment depth, push rod 18 is advanced axially relative to shaft 6 (either by advancing push rod 18, withdrawing shaft 6, or by a combination of these motions) so as to open apart slot sides **12A** and **15A** and urge probe 4 out of slot **13.** After probe 4 is thus disengaged from slot 13, shaft 6 can be further retracted and withdrawn from the tissue together with, or followed by, push rod 18, without drawing either probe **4** or cannula 3 after them thus leaving cannula 3 and probe 10 deployed in the desired position and depth within the patient's body.

It should be noted that the aforementioned sequence of motions may be performed manually by suitable manipulation of the rear portions of shaft 6 and push rod 18, or can be mechanized by any suitable mechanism, as will be clear to one ordinarily skilled in the art.

In a non-limiting exemplary embodiment, insertion tube 6 may be formed from a 26 or 27-gauge, stainless-steel hypodermic needle, suitably processed to form slot 13 and impart the resilient bias described above, while push rod 18 is implemented as a suitably sized pin, typically of similar material, inserted within the lumen of the hollow needle. Typical cannula diameters are, but not limited to, 0.3 to 1.0mm and microdialysis fibers diameters are 0.1mm to 0.4mm.

As noted above, users of automated drug delivery units and metabolite monitoring systems are required to attach the units to the body at points removed from each other to ensure that the metabolite samples are indicative of an overall metabolite content in the body and not the drug rich area surrounding cannula and probe arrangement **2**. The present invention addresses this issue by providing a drug-delivery and metabolite-sampling synchronization scheme having a sampling delay responsive to changes in dosage rates. It is helpful at this point to define several terms used throughout this document.
- "Dosage" refers to quantity of drug to be delivered.
- "Dosing delay" refers to the time interval between drug injections.
- "Dosage rate" refers to the quantity of drug per time unit.
- "Selectable dosage or dosing rate" refers to a drug delivery scheme providing an option to change either the dosage and dosing rate either mechanically or manually.
- "Constant dosage" refers to a drug delivery scheme in which each drug injection is fixed at a constant dosage.
- "Variable dosage" refers to a drug delivery scheme in which each drug injection varies in dosage.
- "Constant dosing rate" refers to a drug delivery scheme in which the time interval between each drug injection is fixed.
- Variable dosing rate" refers to a drug delivery scheme in which the time interval between each drug injection varies.
- "Sampling probe" refers to a metabolite measuring device including, but not limited to, a polymeric microdialysis fiber, an electrochemical probe, a fiber optic sensor, and a fiber-coupled fluorescence affinity sensor.
- "Sampling time" is a probe-dependent entity; in the context of a microdialysis probe, "sampling time" refers to the time in which the metabolite sample is expelled from the probe, in the context of an electrochemical probe "sampling time" refers to the time in which an electrical parameter is measured, and in the context of a fiber optic sensor, it refers to the time a light pulse is applied to the analyte via the fiber optic.

The present invention teaches a sampling delay responsive to all of the above mentioned drug delivery schemes as summarized below:
- Constant dosage
- Constant dosing delay
- Selectable, constant dosage
- Selectable constant dosing delay
- Selectable variable dosage
- Selectable variable dosing delay

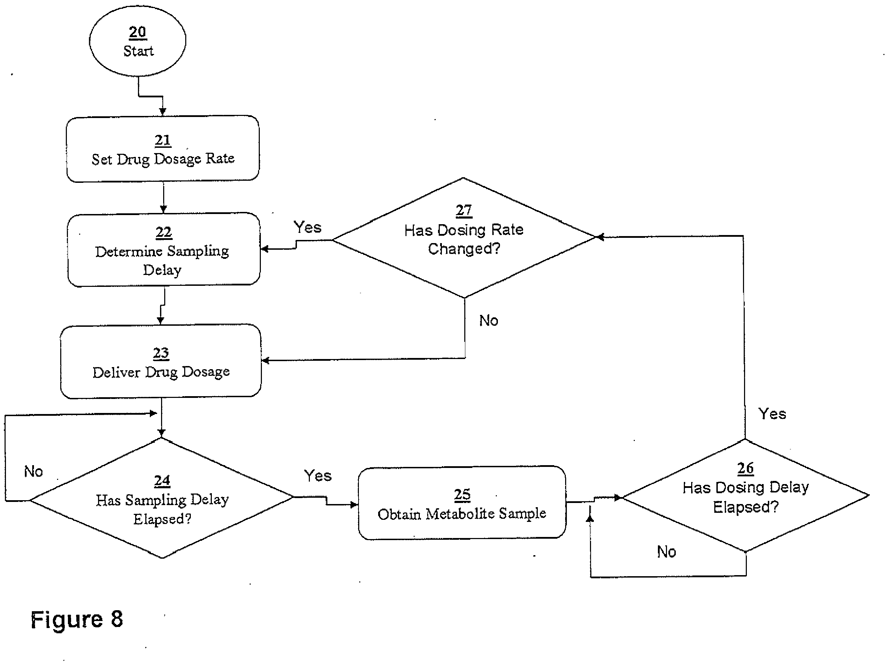

As is known in the art, the amount of time required to disperse or absorb given quantities of a particular drug is a function of physiologic parameters; these parameters serve as the basis for calculation of dosage rates and corresponding sampling delay. In the present invention these values are preset by the manufacturer or manually entered by a knowledgeable practitioner. In a non-limiting, preferred embodiment, an algorithm determines the required sampling delay in direct proportion to the dosage rate as illustrated in the flow chart of Figure 8. Upon initialization (step **20**) a dosage rate is established (step **21**) either automatically based on preset parameters or manually. The system calculates a sampling delay **22** in proportion to the dosage rate that will enable the metabolite sampling to be completed prior to commencement of the next drug delivery at the end of the dosing delay. The system delivers a drug dosage (step **23**). The system verifies that the previously established sampling delay has elapsed (step **24**). If not, the system continues to recheck until the sampling delay has elapsed upon which the system obtains a metabolite sample (step **25**). The system verifies that the established dosing delay has elapsed (step **26**). If not, again the system waits while continuing to recheck until the dosing delay has elapsed. After the dosing delay has elapsed, the system checks if the dosage rate has changed (step **27**). If yes, the system proceeds to recalculate an adjusted sampling delay in proportion to the new dosing rate (step **22**). If the dosing rate remains unchanged the system repeats the steps beginning from step **23**.

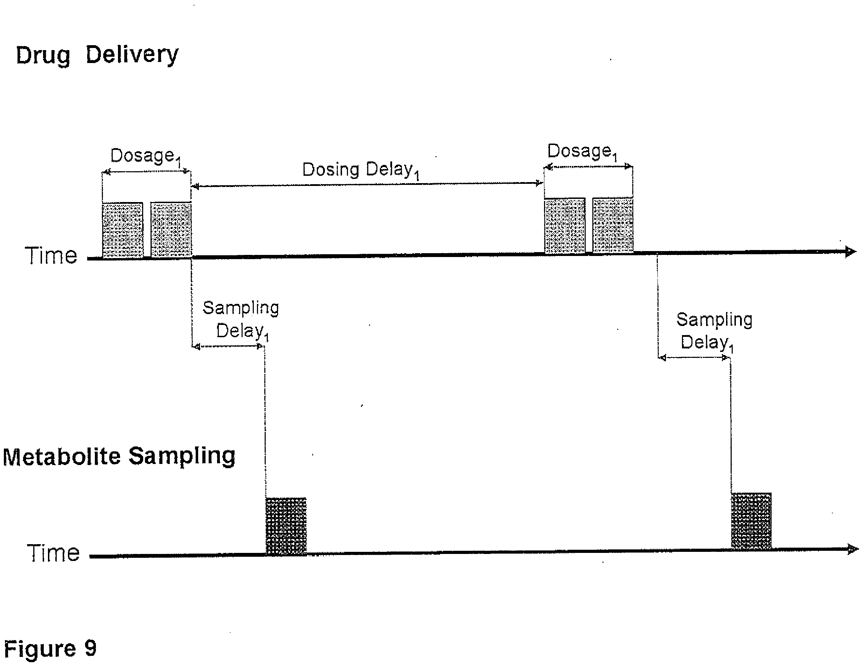

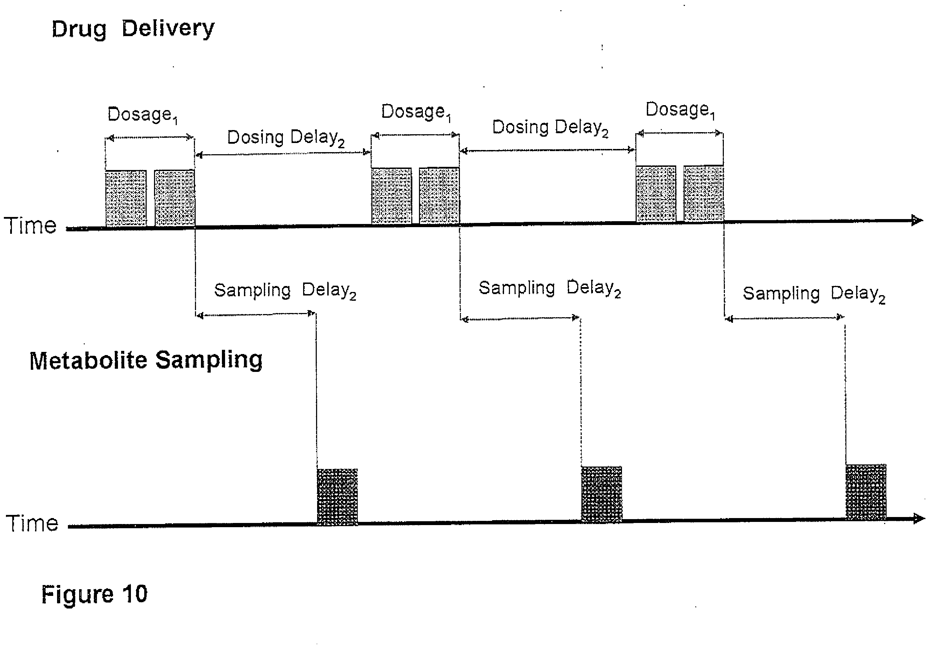

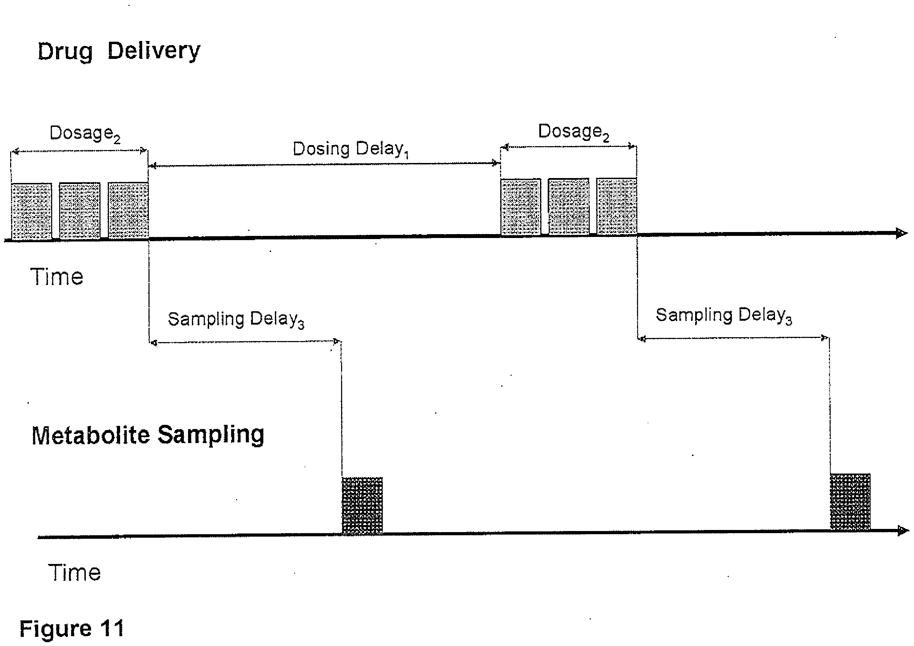

Figure 9 depicts a first delivery and sampling synchronization scheme as a function of time. The system delivers a first double-pulsed Dosage₁, waits until Sampling Delay₁ elapses, obtains a metabolite sample, waits until Dosing Delay₁ elapses, delivers a second double-pulsed Dosage₁, waits until Sampling Delay₁ elapses, and then obtains a second metabolite sample. Figure 10 depicts a second delivery and sampling synchronization scheme in which the dosage rate has increased by decreasing the dosing delay while holding the double-pulsed dosage constant. Accordingly, a longer Sampling Delay₂ is required. Figure 11 depicts an increase in dosage rate from that depicted in Figure 9 by increasing the dosage to triple-pulsed dosages delivered in the same dosing delay of Figure 9. Accordingly, Sampling Delay₃ increases to allow the greater quantity of liquid to be metabolized before obtaining the metabolite sample. As noted above, the present invention includes a similar synchronization scheme when the drug delivery is executed in variable dosages or dosing delays rates thereby advantageously providing useful metabolite monitoring in wide variety of drug delivery options. In a non-limiting embodiment, dosing delays between drug deliveries typically range from 1 dosage/minute-1 dosage/30 minutes and sampling delays typically vary from 1 sample/minute-1 sample/10 minutes. It should be noted that the present invention includes varying sampling schemes in which a plurality of samples are obtained between drug deliveries and/or a sampling frequency that decreases as the system detects metabolite levels that have achieved predefined, steady-state levels.

As noted above, control unit **42** includes processor **44**; applies a synchronization algorithm implemented as software in an exemplary non-limiting embodiment to calculate the dosing and sampling delays; however, should be noted be noted that embedded hardware is included within the scope of the present invention.

The second primary aspect of the present invention addresses complications arising from sudden changes in the flow path diameter as described above. As is known in the art, a dialysate sample enters the optical measuring-cell and mixes with a metabolite specific reagent that changes color in accordance with the content of the metabolite of interest thereby providing a basis for metabolite-indicative transmission data. This particular aspect of the invention is directed at maintaining a uniform flow of a dialysate/reagent mixture in a quantity capable of providing meaningful, metabolite-indicative transmission data. At this point it is helpful to define some additional terms:
- "Transparent" refers to the ability to readily transmit a wavelength of electromagnet radiation of interest sufficiently to allow optical measurement of the changing optical properties of the sample solution.
- "Groove" refers to channel of any depth; whether sufficient to form a through-channel through an entire sheet of material or whether non-penetrating, forming a closed-bottom channel in the sheet of material in which it is disposed.
- "Slot" refers to a through-channel that passes through the entire thickness of the layer of material in which it is disposed.
- "Meandering" refers to geometric arrangement which passes to and fro across a give area.
- "Spiral" refers to any shape of a type colloquially referred to as a "spiral", independent of the exact geometric form, but most typically approximating to at least part of an Archimedean spiral.
- "Dialysate" refers to a solution, typically saline, passed through a microdialysis probe so as to absorb material from the patient's body by way of diffusion through a membrane of the probe.
- "Metabolite" refers to a substance that effects an intermediate or product of metabolism or a body fluid having a metabolic state indicative of a required drug dosage.

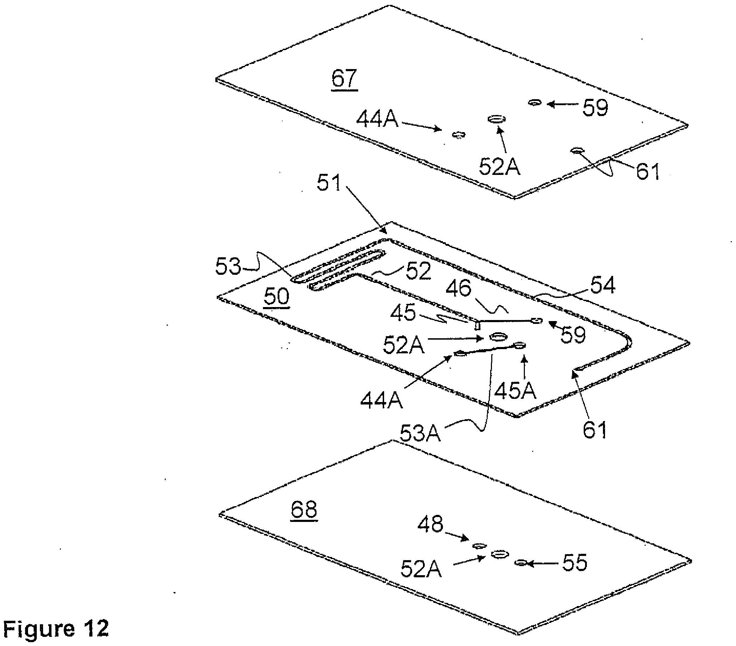

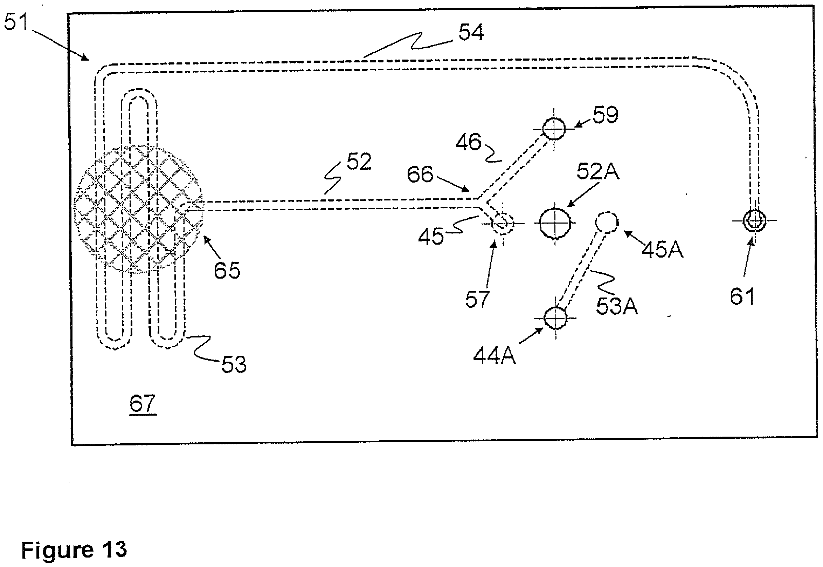

Figure 12 is an isometric, exploded view of a non-limiting, exemplary embodiment of an optical measuring-cell **35** that includes a sheet of material **50** having a primary slot, generally designated as **51**, that defines a flow path for the dialysate/reagent mixture through measuring-cell. Primary slot **51** includes a non-illuminated, mixing segment **52** for facilitating a complete and uniform mixing of reagent and dialysate sample, a non-illuminated drain segment **54** and an illuminated, measuring segment **53** in which optical measurements are obtained. In a non-limiting, preferred embodiment measuring segment **53** is configured in a substantially sinusoidal geometric arrangement thereby providing an illuminated flow path of at least 1.5 times greater, and more preferably at least 2 times greater, and in certain cases at least three times greater that the maximum dimension of an illuminated area **65** (Figure 13). It should be noted that any meandering, spiral, or any other geometrical arrangement maximizing path length while changing path direction gradually are included within the scope of the present invention. These geometrical arrangements advantageously enable a sufficient quantity of sample to be illuminated to produce meaningful transmission data while eliminating the need to change the cross-sectional area of the flow path.

Additional fine slots include a dialysate slot **45** and a reagent slot **46** that intersect with the downstream extremity of mixing segment **52.** A dialysate inlet **57,** most clearly visible in Figure 13, is disposed at the downstream end of the dialysate slot **45** and, similarly, a reagent inlet **59** is disposed at the downstream end of reagent slot **46.** A drain port **61** is disposed at the downstream extremity of drain segment **54.** A cannula-insertion portal **52A** disposed in sheet **50** enabling a cannula inserter mechanism to pass through the measuring-cell into the tissue of a subject as will be discussed. A saline slot **53** is also disposed in sheet **50** and has a saline inlet **44A** disposed at the downstream end and a saline outlet **45A** disposed at the upstream end. Sheet **50** is laminated between a transparent cover sheet **67** and a transparent bottom sheet **68** so as to enclose and to seal primary slot **51,** dialysate slot **45,** reagent slot **46,** and saline slot **53** thereby transforming them into leak-proof, flow channels. Cover sheet 67 includes a corresponding saline inlet **44A,** a corresponding reagent inlet **59,** and a corresponding cannula-insertion portal **52A** and, similarly, bottom sheet **68** includes a corresponding dialysate inlet **48,** saline outlet **55** to the probe (not shown), and a corresponding cannula-insertion portal **52A.**

Figure 13, is a schematic top view of an assembled, optical-measuring-cell depicting hidden flow paths, inlets, and outlets. During operation, saline is injected through saline inlet **44A** into saline flow path **53A** through which the saline exits the measuring-cell through saline outlet **45A** into and enters the probe. Dialysate diffuses through the probe membrane and returns to dialysate inlet **57** into dialysate flow path **45** and continues to the downstream end of mixing segment **52.** Reagent previously injected through reagent inlet **59** into inlet flow path **46** flows downstream to mixing junction **66** where dialysate flow path **45** and reagent flow path **46** intersect. The reduced length of dialysate flow path **45** minimizes the delay in measuring the metabolite content by facilitating almost immediate mixing with the reagent at mixing junction **66.** The dialysate sample continues to mix as it flows through mixing segment **52** so that when the sample enters illumination segment **53** the reagent and metabolite has completely mixed, reacted and achieved the final and color. The mixture is illuminated, in a non-limiting preferred embodiment by infrared or visible light, and the portion of the light passing through the sample is detected by an optical detector and metabolite content derived. The measured sample continues to flow downstream out of illumination segment **53** into drain segment **54** where it exits the measuring-cell via outlet **61** and absorbed by fluid dump **39** (Figure 1). In a non-limiting, exemplary embodiment cover sheet **67** and bottom sheet **68** are constructed from a relatively transparent, polymeric material whereas slotted sheet **50** is constructed from aluminum, polymeric materials, or other durable and bendable materials. It should be noted a middle sheet **50** of any material having a transparency less than cover and bottom sheets **67** and **68** to the electromagnetic radiation employed is included in the scope of the present invention. In a non-limiting, exemplary embodiment; the middle sheet is 0.1-0.3 millimeters thick and the collective volume of all of the flow paths uses no more than about 1 micro-liter of liquid.

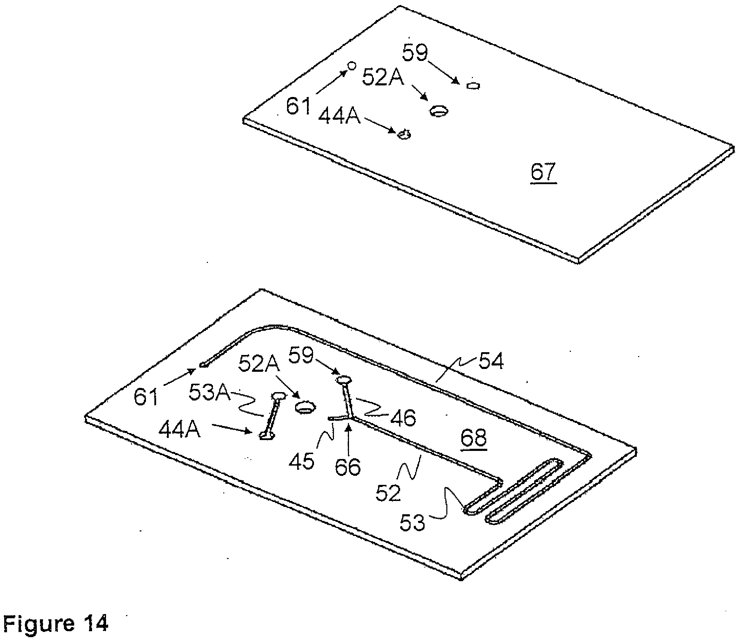

Figures 14-16 depict a second, non-limiting preferred embodiment employing a reflective, optical-measuring scheme by way of a transparent, cover sheet **67** bonded to a sheet of at least partially relatively reflective material **68** as will be discussed. This two-layered measuring cell has a flow path configuration analogous to the one described above; however, in the present embodiment the flow paths are defined by fine groves as opposed to slots as described above.

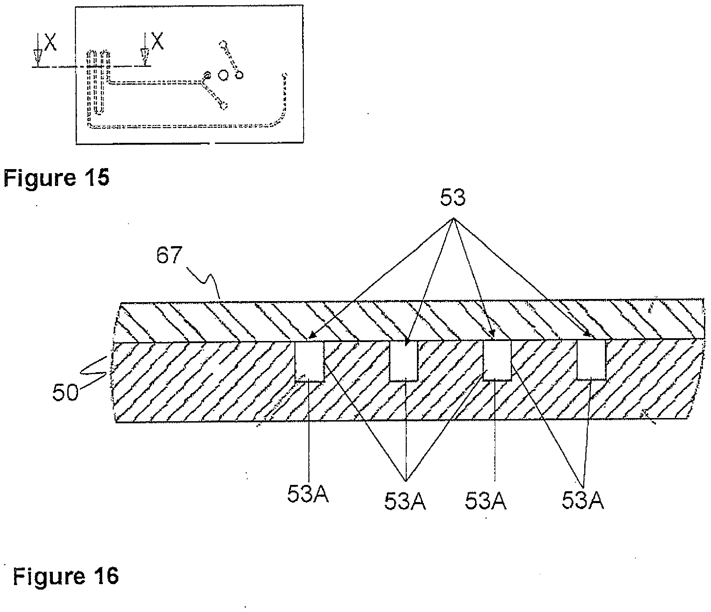

Figure 16 is a cross-sectional view of the measuring-cell along line X-X of Figure 15 and depicts the multiple flow paths of the illumination segment **53** meandering through illumination area **65** (most clearly shown in Figure 13). Reflective surfaces **53A** lining the inner surface of groove segment **53** reflect illumination to optical sensor **40** (Figure 1). Accordingly, for the purposes of this document, "reflective sheet" refers to a sheet having reflective surfaces **53A** lining flow path **53** even when the majority sheet **50** is non-reflective. In a non-limiting embodiment, reflective groove surfaces 53A are implemented by way of polished groove surfaces disposed in sheet **50** constructed from a metallic material. As noted above, the meandering flow path through illumination region **65** advantageously enables a sufficient quantity of dialysate/reagent mixture to be exposed to an illumination source for the sake of collecting optical data sample to without changing the cross-sectional area of the flow path throughout the measuring-cell. It should be noted that embodiments have a primary flow path gradually changing in cross-sectional area, but not exceeding twenty percent between any two points along the primary flow path **51,** are included within the scope of the present invention.

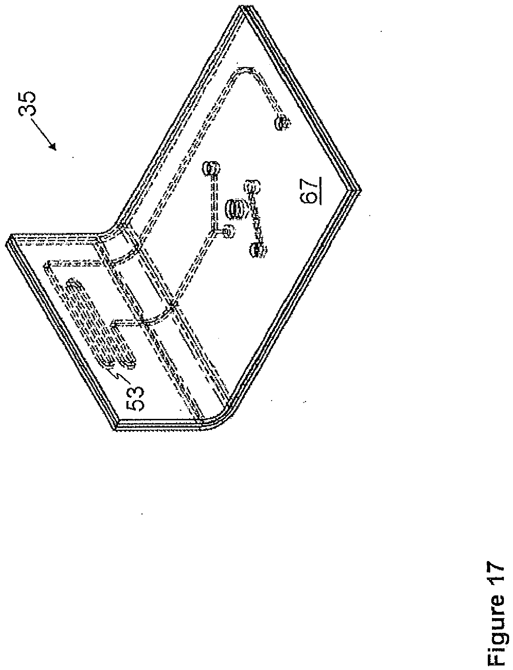

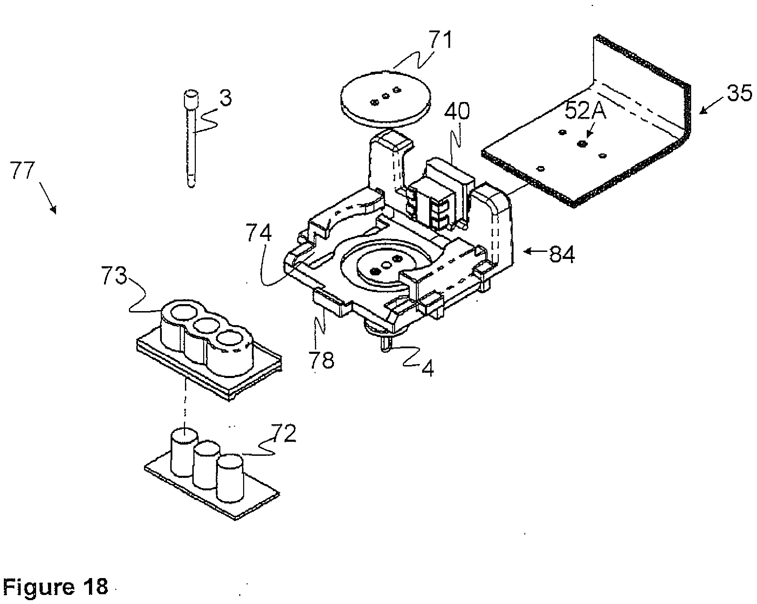

Figure 17 depicts a non-limiting exemplary embodiments of laminated optical-measuring-cell **35** in a bent, deployment state in which a first portion is substantially perpendicular to a second portion so as to align illumination segment **53** with optical sensor **40** (Figure 18) when deployed in system housing as will be discussed. It should also be noted that laminated, optical-measuring-cells **35** bent into deployment states of non-perpendicular angles are also included within the state of the present invention. It should be further appreciated that either laminated optical-measuring-cell **69** or individual sheets of material bent manually, by way of the system housing, or by way of a specialized bending tool are all included in the scope of the present invention.

Referring to Figure **18****,** optical measuring-cell **35** is deployed in system housing **84** by inserting cell **35** through housing slot (not shown) into parallel channels **74.** A bottom sealing disk **71** is seated in the bottom of housing **70** and forms a seal between probe **4** and measuring-cell **69** as will be discussed. A multiple-port structure, generally designated **77,** includes a silicon port structure **72** and a corresponding silicon port housing **73** of hard plastic or equivalent material. Silicon port structure **72** includes a series of three hollow, silicon plugs integrally formed with a silicon plate. Deployment involves loading silicon port structure **72** into silicon port housing **73** and sliding the resulting multiple-port structure **77** into parallel channels **74** disposed in system housing **70** on top of previously deployed measuring-cell **35.** The collective thickness of optical measuring-cell **35** and multiple-port structure **72** secures each of them in place. Proper alignment is achieved by way of stoppers (measuring-cell stopper **78** and multiple port structure stopper, not shown) that define a maximum insertion distance in which measuring-cell **35** multiple port structure **77** are in proper alignment with each other, and measuring-cell **35** is also in proper alignment with dialysis probe **4** and optical detector **40.** The modular nature of system **1** facilitates convenient replacement of measuring-cell **35** and multiple-port structure **77.**

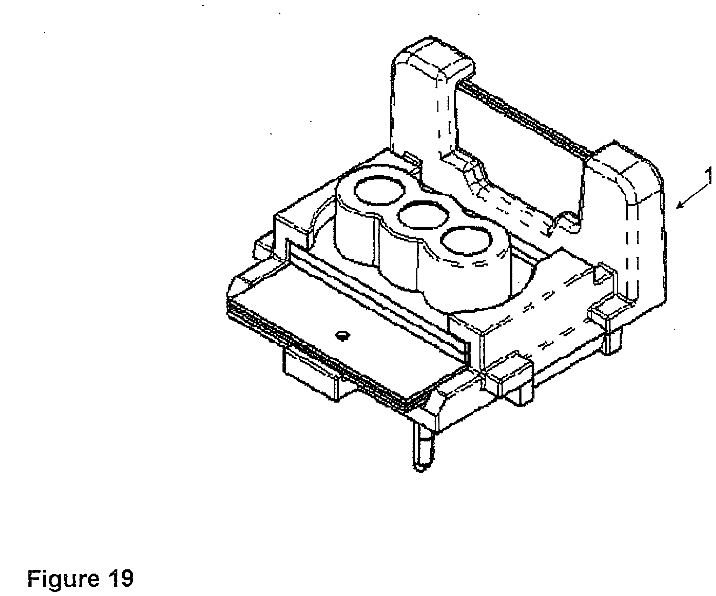

Figure 19 depicts a combination drug-delivery and metabolite monitoring system **1** with the deployed optical measuring-cell **35** and multiple-port structure **77.**

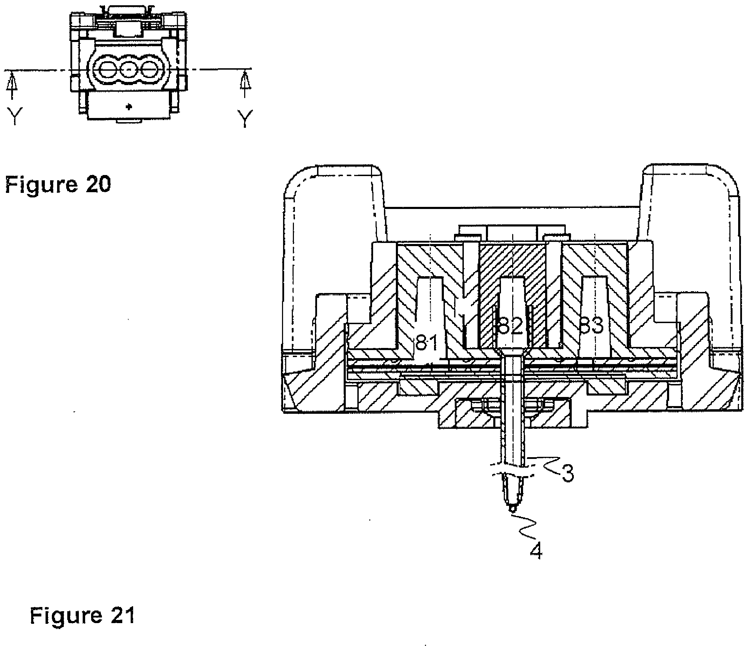

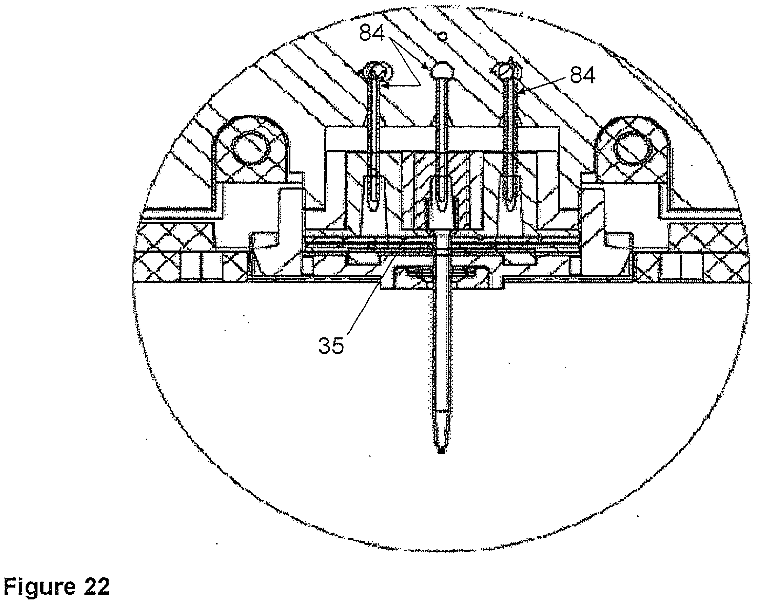

Figures 20-22 depict a non-limiting, exemplary interface between system liquid inlets and separate, pressurized reservoirs containing saline, reagent and drug (not shown). As mentioned above, multiple-port structure 77 includes a silicon port structure 72 having a series of three hollow, silicon plugs integrally formed with a silicon plate. In non-limiting exemplary embodiment silicon port **81** feeds reagent inlet **59,** silicon port **82** feeds cannula **3,** and silicon port **83** feeds saline inlet **44A.** A needle **84** disposed in each port defines a flow path between each reservoir and the appropriate port. The cavity in each port facilitates loading of the desired liquid into the port before entry into the corresponding inlet. It should be noted that in a non-limiting, exemplary embodiment, all sealing elements are constructed from silicone; however, it should be appreciate that any material providing the functionality associated with silicon is.also

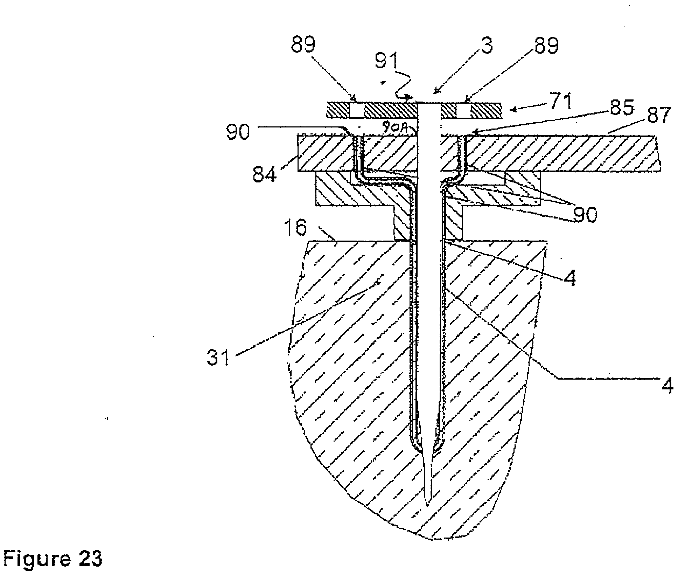

Figure 23 depicts microdialysis probe **4** disposed in tissue **31** and leak-proof connection to system housing base **84.** Probe **4** is formed by inserting opposite ends of a hollow fiber segment **4** through two holes **85** in the housing base **84,** gluing each end with adhesive **90** to housing base **84,** and severing any remaining fiber protruding from housing holes **85** so the fiber ends are flush with inner surface **87** of housing base **84.** Silicon seal **71,** having a triple perforation **89,** is disposed on inner housing surface **87** with outer perforations **89** in alignment with the probe ends and center perforation **91** in alignment with a cannula opening **90A** disposed in housing **84.** Seal **71** is pressed against inner housing surface **87** by deployed measuring-cell 35 and multiple port structure **77** thereby forming a leak-proof flow path between probe openings with measuring-cell inlet and outlet with cannula **3** disposed in close proximity.

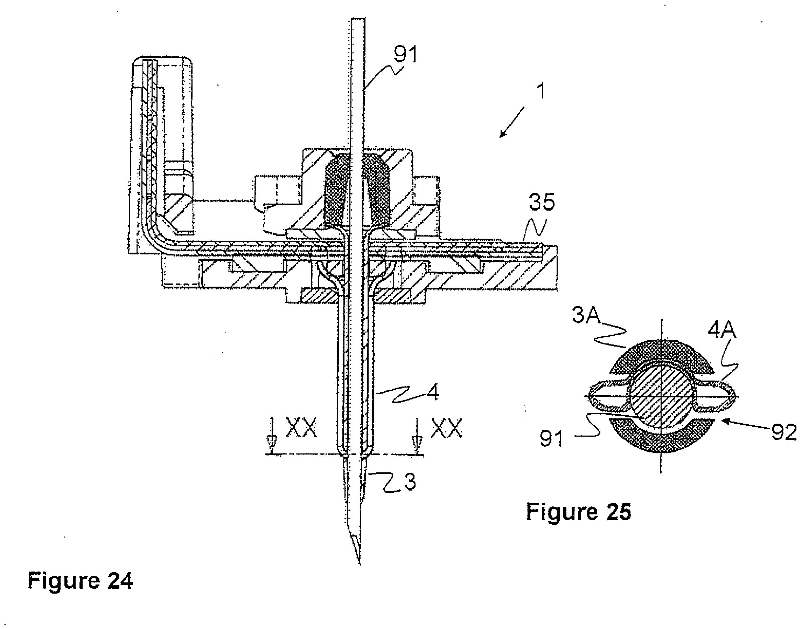

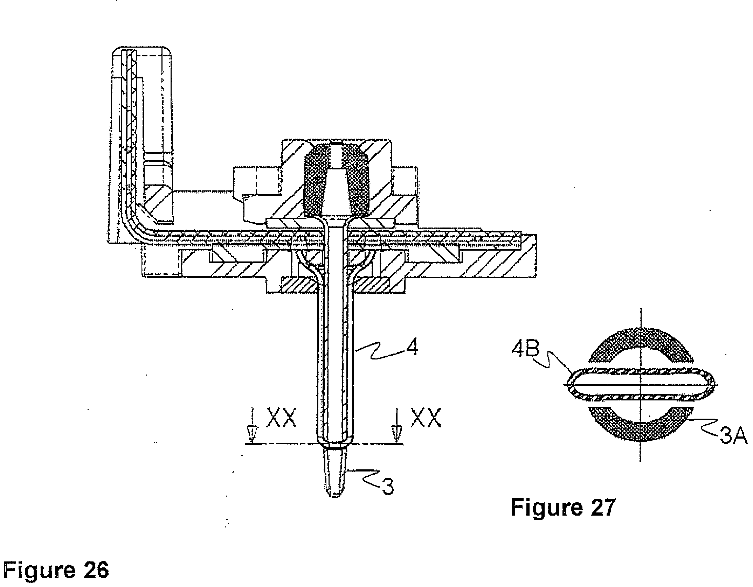

Figures 24-27 depict an alternative embodiment of a cannula/microdialysis probe arrangement in which sampling probe **4,** implemented as a microdialysis fiber, is threaded through a set of perforations 92 in cannula **wall 3A.** Figure 25 depicts probe **4** in a compressed deployment state while cannula-inserter **91** is disposed in the cannula lumen whereas Figure 27 depicts probe **4** after resuming a natural non-compressed state after cannula-inserter **91** has been removed from the cannula lumen.

The present invention has particular relevance in regards to insulin delivery and glucose monitoring; however, it should be appreciated that it has relevance to any combination drug-delivery system including monitoring either the resulting metabolic state of a body fluid or direct monitoring of the delivered drug for the sake of maintaining a desired metabolic state.

The metabolite-monitoring system and its various components may be constructed from any suitable materials including, but not limited to, polymeric materials and metallic materials as is known in the art.

It will be appreciated that the above descriptions are intended only to serve as examples, and that many other embodiments are possible within the scope of the present invention as defined in the appended claims.

## Claims

1. A layered, optical measuring-cell (35) partially illuminated by a light source for a metabolite-monitoring unit comprising:
(a) a sheet (50) of material having a groove defining an illuminated flow-path segment (53) in fluid connection with a non-illuminated flow-path segment (52, 54); and
(b) at least one sheet (67, 68) of transparent material bonded to said sheet (50) of material so as to enclose said flow path segments (52,53,54),
wherein a cross-sectional area of said illuminated flow-path segment (53) substantially matches a cross-sectional area of said non-illuminated flow-path segment (52, 54) thereby facilitating uniform flow from said non-illuminated, flow-path segment to said illuminated, flow-path segment.

2. The layered, optical measuring-cell of claim 1, wherein said illuminated flow-path segment (53) has a geometrical arrangement.of a length at least 1.5 times greater than a maximum dimension of an area (65) of sheet (50) illuminated by the light source.

3. The optical measuring-cell of claim 1, wherein said illuminated flow-path segment (53) has a geometrical arrangement of a length at least two times greater than a maximum dimension of an area (65) of sheet (50) illuminated by the light source.

4. The optical measuring-cell of claim 1, wherein said illuminated flow path-segment (53) has a geometrical arrangement of a length at least three times greater than a maximum dimension of an area (65) of sheet (50) illuminated by the light source.

5. The layered optical measuring-cell of any of claims 2-4, wherein said geometric arrangement includes a plurality of curves.

6. The optical measuring-cell of any of claims 2-4, wherein said geometric arrangement includes a spiral.

7. The layered optical measuring-cell of any preceding claim, wherein the layered optical measuring cell (35) is bent such that said illuminated flow-path segment (53) is disposed in a first plane and said non-illuminated flow-path segment (52, 54) is disposed in a second plane wherein said first plane is angled relative to said second plane.

8. The optical measuring-cell of any preceding claim, wherein said at least one sheet of transparent material is implemented as two sheets (67, 68), said two sheets being bonded to opposing faces of said sheet (50) wherein said groove defining the flow path segments (52, 53, 54) is at least partially implemented as a slot.

9. The optical measuring-cell of claim 1, wherein said flow path segments (52, 53, 54) are part of a flow path having a substantially constant cross-sectional area not varying more than 20% between any two points along the length of said flow path.

10. The optical measuring-cell of claim 1, wherein said sheet (50) of material is less transparent than said at least one sheet (67, 68) of transparent material.

11. The optical measuring-cell of claim 1, wherein said non-illuminated flow path segment (52) defines a flow path upstream from an area (65) illuminated by said light source for mixing of dialysate and reagent prior to exposure to said light source.

12. A metabolite monitoring system comprising:
(a) the optical measuring cell (35) of any preceding claim; and
(b) a hollow fiber microdialysis probe (4),
wherein an end of said hollow fiber microdialysis probe (4) is sealed against an inlet of optical measuring cell (35).

13. A metabolite monitoring system comprising:
(a) the optical measuring cell (35) of any preceding claim; and
(b) an optical sensor (40) deployed for sensing a color change in liquid within said illuminated flow path segment (53).
